(19)

Europäisches
Patentamt
European
Patent Office
Office européen
des brevets

(11)  **EP 2 394 696 A1**

(12)  **EUROPEAN PATENT APPLICATION**

(43)  Date of publication:
**14.12.2011 Bulletin 2011/50**

(51)  Int Cl.:
*A61N 1/36* (2006.01)        *A61N 1/05* (2006.01)

(21)  Application number: **10305609.9**

(22)  Date of filing: **09.06.2010**

(84)  Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71)  Applicants:
• **Centre National de la Recherche Scientifique (CNRS)**
**75016 Paris (FR)**
• **Université de Bordeaux 1**
**33400 Talence (FR)**

(72)  Inventors:
• **Yvert, Blaise**
**33360 Carignan de Berdeaux (FR)**

• **Mazzocco, Claire**
**33410 Cardan (FR)**
• **Rousseau, Lionel**
**94170 Le Perreux Sur Marne (FR)**
• **Urbanova, Veronika**
**54371 Hostinne (CZ)**
• **Heim, Matthias**
**33000 Bordeaux (FR)**

(74)  Representative: **Roussel, Eric Marcel Henri et al**
**Cabinet Regimbeau**
**Espace Performance**
**Bâtiment K**
**35769 Saint-Grégoire (FR)**

(54)  **Device to stimulate or record a signal to or from a living tissue**

(57)  The invention concerns a device to stimulate or record a signal to or from a living tissue, comprising at least one electrically conductive first electrode comprising a zone for application to the living tissue and a conductor for sending or receiving a signal voltage to or from the zone.

According to the invention, the zone for application to the living tissue has a first geometric surface formed of an external porous layer consisting in a second material (M2por) being porous and arranged in such a manner that the first electrode has a standard deviation of intrinsic recording voltage noise $N_{M2por} \leq 0.9\ N_{M2}$,
wherein $N_{M2}$ is the standard deviation of the intrinsic recording voltage noise measured with another second comparison electrode being the same as the first electrode but having its zone having the same first geometric surface formed of said second material (M2) being non porous.

**FIG. 8**

EP 2 394 696 A1

## Description

The invention concerns a device to stimulate or record a signal to or from a living tissue, comprising at least one electrically conductive first electrode and/or supplementary conductive surface comprising a zone for application to the living tissue and a conductor for sending or receiving a signal voltage to or from the zone, wherein the zone for application to the living tissue is connected to said conductor.

The present invention solves issues encountered when recording or stimulating neural networks using metal electrodes, and especially using microelectrodes.

A current challenge is to build efficient microelectrode arrays (MEAs) for recording and electrically stimulating electrogenic cells (neurons, glial cells, muscular cells, cardiac cells, stem cells,). The present invention is illustrated for the case of neurons but equally applies to any electrogenic cells other than neurons.

The stake of neural stimulation device is to build neural prosthesis or implants to compensate function loss due to a lesion or the degeneration of a part of the Central Nervous System (CNS). For this purpose, two main approaches are considered: macrostimulation using macroscopic electrodes or macroelectrodes (surface of several mum), or microstimulation using microelectrodes (below several hundreds of $(\mu m)^2$). Macroscopic stimulation of the brain is used in routine to suppress tremors in patients suffering from Parkinson diseases, while macrostimulation of the spinal cord alleviates chronic neuropathic pain. More recently, microstimulation has gained increasing interest to achieve more focal and specialized stimulations of restricted groups of neurons. This is of particular interest for the development of sensory neural prosthesis such as cochlear, retinal, subcortical, or cortical implants.

As described in patent application WO 2009/053333, the focality of an electrical stimulation can be improved by introducing a supplementary conductive surface (also called ground surface when connected to the ground) surrounding the electrodes of the array. The focality is all the higher that the surface conductance of the supplementary conductive surface is high.

In order to achieve high surface conductance, microelectrodes coated with black platinum have been developed. However, black platinum is fragile, making these electrodes not stable along time.

One problem of those devices applied to a living tissue is that they should be able to record signals of very low amplitude from, and/or send sufficiently high electric currents due to the application to a living tissue. It is wished to send sufficiently high signals to the living tissue or to receive very small signals from the living tissue.

Thus, recording noise and current injection capabilities should be improved.

The goal of the invention is to have a device enabling to send sufficiently high or receive very small electric signals to or from a living tissue.

According to the invention, the surface of the zone for application to the living tissue is modified by an external porous layer.

According to the invention, the surface of the zone for application to the living tissue has an external porous modified layer to have an intrinsic recording voltage noise at least 10% smaller than that achieved with the same but not porous material.

According to the invention of claim 1, the zone for application to the living tissue belonging to the first electrode and/or supplementary conductive surface has a first geometric surface formed of an external porous layer consisting in at least one second material being porous and arranged in such a manner that the first electrode has a standard deviation of intrinsic recording voltage noise $N_{M2por}$ measured under defined conditions according to the following first formula

$$N_{M2por} \leq 0.9\, N_{M2} ,$$

wherein $N_{M2}$ is the standard deviation of the intrinsic recording voltage noise measured under the same defined conditions with another second comparison electrode and/or supplementary conductive surface being the same as the first electrode having its zone for application to the living tissue having the same first geometric surface but formed of said at least one second material (M2) being non porous,

wherein said conditions comprise measuring the voltage in a bandwith between 1 and 3000 Hz in a physiological liquid at 25 °C.

The inventors have discovered that macroporous or mesoporous modification of electrodes lead to a reduction of noise level by at least 10% with respect to the same flat material, or to a reduction of noise level according to the first formula. The second comparison electrode does not belong to the device according to the invention.

According to an embodiment, $N_{M2por} \leq 0.75\, N_{M2}$.

According to another embodiment, $N_{M2por} \leq 0.5\, N_{M2}$.

According to an embodiment of the invention, according to the second following formula :

$$\log (N_{M2por}) \leq 1.5 - 0.57 \log (D),$$
wherein
log is the decimal logarithm,
D is a width of the zone of the first electrode in $\mu$m,
N is in $\mu$V.

In an embodiment, the physiological liquid is composed of (in mM): 113 NaCl, 4.5 KC1, 2 CaCl$_2$2H$_2$O, 1 MgCl$_2$6H$_2$O, 25 NaHCO$_3$, 1 NaH$_2$PO$_4$H$_2$O and 11 D-Glucose.

Noise reduction depends on the width or diameter of microelectrode.

One critical parameter of electrodes used to record or stimulate the CNS is the surface of contact with the extracellular conductive medium (active surface). A large active surface leads to better performances for recording and stimulation:

Regarding recording, a low intrinsic noise level of the electrode is obtained.

Regarding electrical stimulation, the capacity to inject higher currents through the electrode without damage of the electrode and the tissue due to electrochemical reaction at the interface.

The other claims concern embodiments of the invention.

In an embodiment,

$$\log (N_{M2por} \leq 1.5 - 0.6 \log (D),$$
wherein
log is the decimal logarithm,
D is a width of the zone of the first electrode in $\mu$m,
N is in $\mu$V.

The invention will be better understood by reference to the appended drawings showing embodiments of the invention

The invention will be better understood on reading the following description given solely as a non-limiting example with reference to the appended drawings in which:

- Figures 1A, 1B, 1C, 1D, 1E, IF and 1G are schematic view of examples of configurations of a set of electrodes according to the invention.
- Figure 1H is a schematic view of a set of miucroelectrodes surrounded by a supplementary conductive surface;
- Figure 2A is a microscopic photo of an example of a macroporous planar disk microelectrode;
- Figure 2B is a microscopic photo of a macroporous 3D conical microelectrode (B);
- Figure 3 is a microscopic photo of an example of a mesoporous planar disk microelectrode;
- Figure 4 shows a typical cyclic voltamogram of a gold microelectrode before (interrupted line) and after macroporous modification (continuous line);
- Figure 5 shows a typical cyclic voltamogram of a platinum microelectrode before (interrupted line) and after mesoporous modification (continuous line);
- Figures 6A and 6B show an example of noise reduction obtained with a macroporous microelectrode;
- Figures 7A and 7B show an example of noise reduction following mesoporous modification;
- Figure 8 shows noise reduction obtained with mesoporous modification for 5 microelectrodes of different diameters;
- Figure 9 shows example of action potentials recorded using mesoporous microelectrodes;
- Figure 10 shows example of a local field potential recorded using mesoporous microelectrodes.

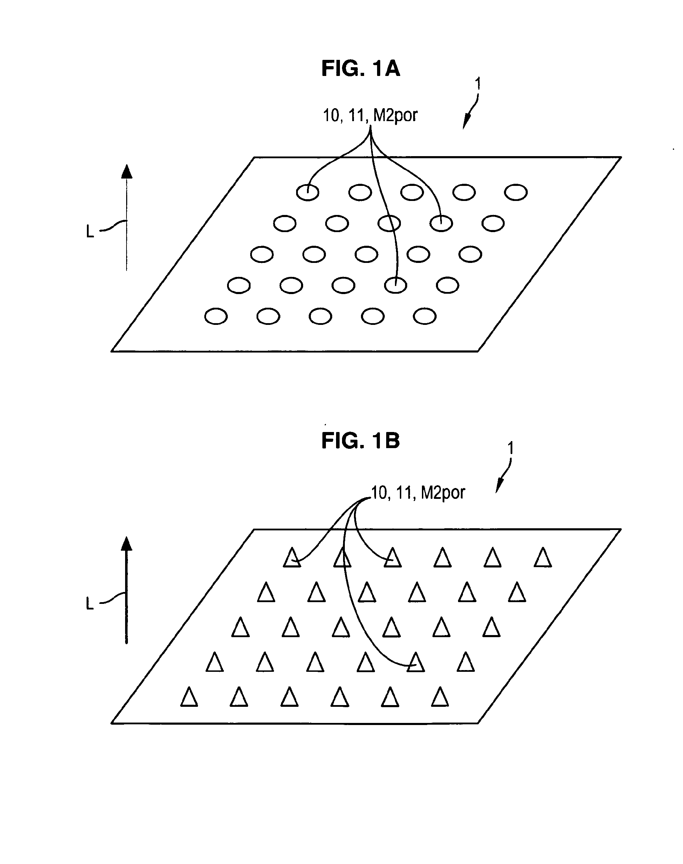

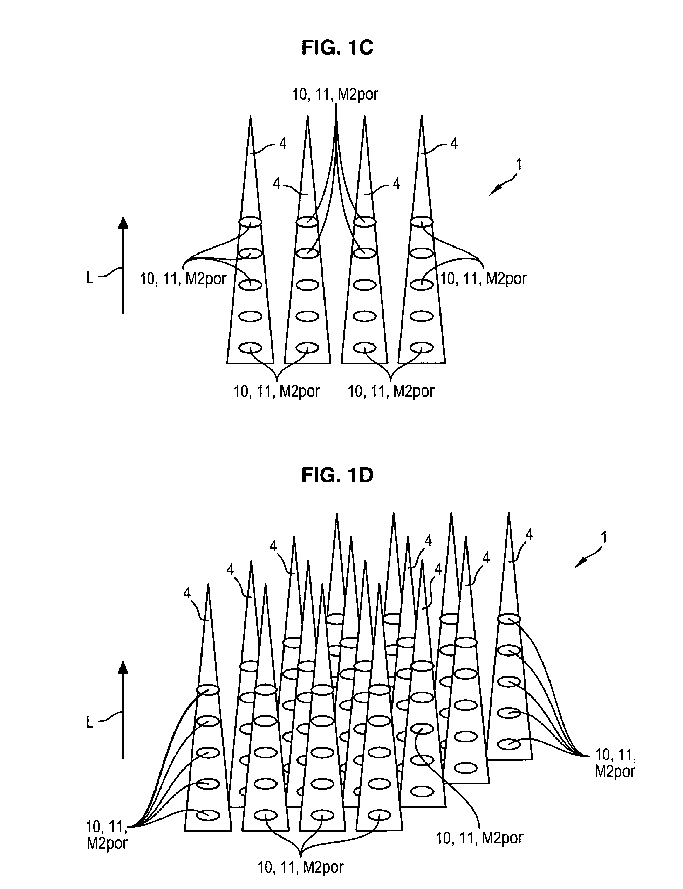

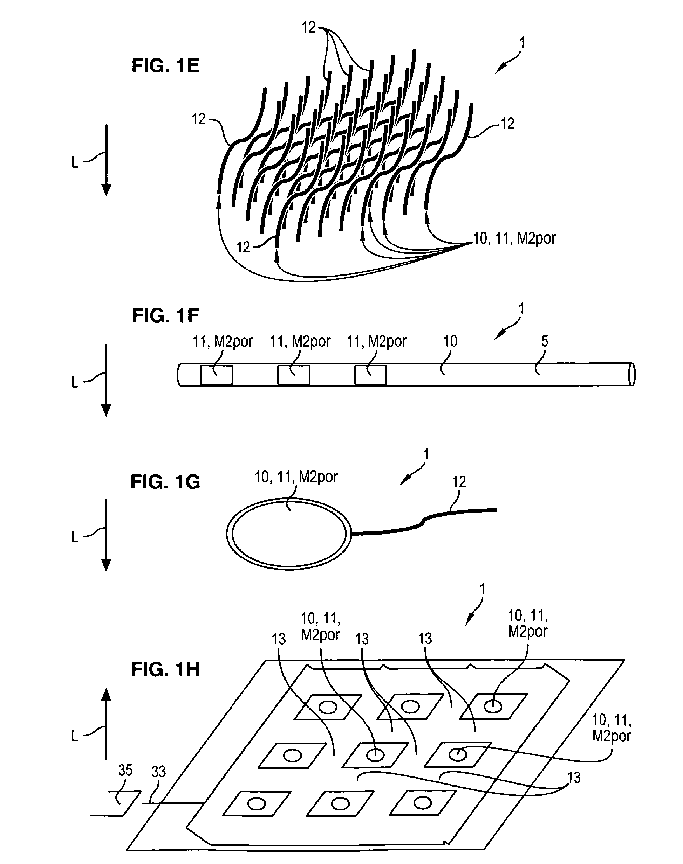

In the drawings, each first electrode 10 is electrically-conductive and has a zone 11 for application to a living tissue, which is connected to a conductor for sending a signal voltage to the living tissue in order to stimulate the living tissue or for receiving a signal voltage from the living tissue in order to record a signal from the living tissue. The zone 11 is for example situated at the end of the conductor. The device can comprise a single electrode 10 or a plurality of electrodes 10. The electrode 10 can be a microelectrode 10, for example as in figures 1A, 1B, 1C, 1D, 1E and IF or a macroelectrode 10 for example as in figure 1G, or a supplementary conductive surface as in Figure 1H.

For example, the device to stimulate a living tissue comprises an array 1 of electrodes 10 arranged in a determined configuration side—by—side, each electrode 10 comprising a zone 11 for local application to the living tissue and a conductor for sending a stimulation signal, wherein each electrode 10 can be selected for application of an electric stimulation signal or the reception of a signal from the living tissue, the electrodes 10 being insulated from each other, the conductors being insulated from each other, the zones 11 being insulated from each other, as in examples of Figures 1A, 1B, 1C, 1D, 1E and 1F.

In an embodiment, the present invention consists in using 2D or 3D microelectrodes with a small geometric surface (typically for example 1 to 10000 $(\mu m)^2$) but showing a high active macroporous or mesoporous surface to record or stimulate electrogenic cells. This is further illustrated in the case of neurons, but can equally apply to any other electrogenic cells, such as glial cells, muscular cells, cardiac cells, or stem cells. Moreover the present invention is illustrated in the case of microelectrodes for which it is expected to bring important benefits. However, the invention also applies to macroelectrodes. Also, the present invention is illustrated for microelectrode arrays obtained by microfabrication (e.g., lithography) but can equally apply to isolated microwires or arrays of microwires, the non-isolated tips of which being used as a microelectrode, as in example of Figure 1E. Different examples of electrodes and arrays of electrodes for which the invention applies are illustrated in Figures 1A, 1B, 1C, 1D, E , IF and 1G. Of course the invention applies also to a single electrode or a set of electrodes or an array of electrodes with regular or irregular spacing and even positions in different planes in 3D.

In the embodiment of Figure 1A, each microelectrode 10 of an array 1 of microelectrodes has a zone 11 for application to a living tissue, which has a disk-shaped planar external surface and which is connected to a conductor for sending a signal voltage to the living tissue in order to stimulate the living tissue or for receiving a signal voltage from the living tissue in order to record a signal from the living tissue. For example, the zone 11 is transverse to the direction L of application against the living tissue. Of course, the zone 11 can have a planar or not planar (3D) external surface not being disk-shaped.

In Figure 1H, a supplementary third conductive surface 3 for application also against the living tissue is provided in the vicinity of the zone(s) 11. The supplementary conductive surface 3 is isolated from the zones 11. The supplementary conductive surface 3 is for example in Figures 1H in the form of a grid. The supplementary conductive surface 3 can be also for example in the form of a plan or any other shape surrounding the electrodes. The supplementary conductive surface 3 comprise a plurality of conductive third zones 13 being located, respectively for a determined plurality of electrodes 10, in the vicinity of the plurality of first zones 11 of said determined electrodes 10 of the array and are insulated from these zones 10. Said plurality of conductive zones 13 belongs to the conductive surface 3 supplemental to the electrodes 10 and separate from the electrodes 10, the supplemental conductive surface 3 being used for all or part of the application against the living tissue. Connection means are provided to ensure electric connection between said plurality of conductive zones 13 of the supplemental surface 3 for all said determined plurality of electrodes 10 insulated from one another, so that the conductive zones 13 of the supplemental surface 3 are substantially equipotential. The supplemental conductive surface 3 is also connected to at least one port intended to be connected to an external return conductor of the signal and being formed to ensure focal stimulation from at least one of the determined plurality of electrodes 10 and to act as common focusing means for several application zones 11 of different determined electrodes 10. The surface 3 is described in document WO 2009/053333 which is incorporated by reference.

In the embodiment of Figure 1B, each zone 11 for application to a living tissue has an external surface in the form of a tip, which is for example conical (shown by a triangle on Figure 1B).

In the embodiment of Figure 1C, a plurality of zones 11 are disposed along the application length L of an insulating substrate 4, wherein the length L is directed towards the living tissue and the substrate has a form narrowing towards the living tissue, for example in the form of a tip. Depending of how deep each substrate 4 will penetrate the living tissue, a different number of zones 11 will be in contact with the living tissue. A multiplicity of substrates 4 carrying each one or a plurality of zones 11 can be provided. Since the substrate can be provided along two first and second dimensions and the zones 11 in the third dimension L perpendicular to the two first and second dimensions, a three dimensional array of electrodes is provided. In figure 1C, one first dimension is provided to form a linear shank of substrates 4. In Figure 1D, the two first and second dimensions are provided.

In the embodiment of Figure 1E, each zone 11 for application to a living tissue has an external surface which is formed by the end of a wire 12, wherein the wire 12 serves as conductor for sending a signal voltage to the living tissue in order to stimulate the living tissue or for receiving a signal voltage from the living tissue in order to record a signal from the living tissue.

In the embodiment of Figure 1F, each zone 11 for application to a living tissue has an external surface which is cylindrical along an insulating tube 5, wherein the zones 11 form part of the external surface of the tube 5 and are disposed one behind the other along the tube 5.

In the embodiment of Figure 1G, the zone 11 for application is of a single macroelectrode and is for example disk-shaped, for example for EEG scalp recording and is connected to said conductor 12.

According to the invention, the zone 11 for application to the living tissue belonging to the first electrode 10 has a first geometric surface formed of an external porous layer consisting in at least one second material $M2por$ being porous and arranged in such a manner that the first electrode has a standard deviation of intrinsic recording voltage noise $N_{M2por}$ measured under defined conditions according to the following first formula

$$N_{M2por} \leq 0.9\, N_{M2}\,,$$

wherein $N_{M2}$ is the standard deviation of the intrinsic recording voltage noise measured under the same defined conditions with another second comparison electrode being the same as the first electrode 10 but having its zone 11 for application to the living tissue having the same first geometric surface formed of said at least one second material M2 being non porous.

For example, the external porous layer consisting in said at least one second material M2por being porous of the first geometric surface of the first electrode 10 is formed on a first underlying material M1 of the zone 11 for application to the living tissue, wherein the first underlying material M1 is connected to said conductor.

Porous zones 11 of metal electrodes 10 or of supplementary conductive surface 3 consisting in M2por are obtained for example by modification of their surface so as to increase the active surface of their application zone 11 by several orders of magnitude without noticeably changing their geometrical surface. This is achieved by electrodeposition of a metal or metals, semiconductors, alloys, conducting polymers or other conducting materials M2por through a template that will define the porosity of the resulting surface.

After electrodeposition, the underlying material M1 may be possibly removed leaving only the porous layer of M2por forming the zone 11 for application to the living tissue. The deposited surface M2por may have a typical thickness of several tens or hundreds of nanometers to a few microns. The geometric surface of the zone 11 is the surface on which the porous external layer is formed and is called geometric, since its geometric area can be calculated from the overall dimensions of the zone 11. For example, in case of figures 1A, 1C, 1D, 1E and 1G, the geometric surface of zone 11 is a disk having a known predetermined diameter D and a corresponding geometric surface area equal to $\pi.D^2/4$. In case of figure 1B, the geometric surface of zone 11 is a cone of known predetermined angle. In case of figure 1F, the geometric surface of zone 11 is a cylinder of known dimensions. The geometric surface of the zone 11 is called first geometric surface and can have one or several first underlying material(s) M1.

The external porous layer on the first geometric surface of the zone 11 for application to the living tissue is formed of one or several second porous material(s) M2por, which can be the same as the first material(s) M1 or can be different from the first material(s) M1. Consequently in the electrode according to the invention, called first electrode, the second porous material(s) M2por is on the first material(s) M1.

The fabrication of the porous layer M2por uses salts or molecules as precursors for the electroformation of the final porous conducting structure. Therefore the toxicity is highly dependent on the type of precursor employed. For example, salts based on platinum and gold which are both noble metals and therefore not toxic themselves are used. The surfactants that are employed to generate the porosity, like octaethyleneglycol or similar molecules, do not present a significant toxicity because in practice the surfactant is washed out after deposition.

In an embodiment, the zone 11 for application to the living tissue is metallic or an alloy or semiconductor or polymer or any other conducting material and the zone 11 for application to the living tissue is made of an underlying material M1 being metallic or an alloy or semiconductor or polymer or any other conducting material, with the second porous external layer made of a material M2por being also metallic or alloy or semiconductor or polymer or any other conducting material on the zone 11.

In an embodiment, the second porous external layer is of the same or different metallic material or materials M2por as the underlying material M1 of the zone 11 for application to the living tissue.

In an example, the second porous external layer and the first underlying material of the first geometric surface of zone 11 for application to the living tissue are made of platinum, i.e.:

$$M1 = Pt \text{ and } M2por = Pt.$$

In another example, the second porous external layer and the first underlying material of the first geometric surface of zone 11 for application to the living tissue are made of gold, i.e.:

$$M1 = Au \text{ and } M2por = Au.$$

In the frame of the current invention, two types of porous structures of M2por are considered:

Macroporous electrodes obtained by electrodeposition of a metal, semiconductor, alloy, conducting polymer or other conducting materials through a colloidal crystal, typically made of latex beads.

Mesoporous electrodes obtained by electrodeposition of a metal, semiconductor, alloy, conducting polymer or other conducting materials from a mixture as described in previous patent US6503382.

The obtained porous layer generally has a uniform pore size, but may also have pores of different sizes.

The obtained porous first microelectrodes are very stable over time, unlike platinum black electrodes which are not considered porous but rough and are very fragile and friable and do not last a long time.

The following results were achieved:

Fabrication of macroporous and microporous first microelectrodes

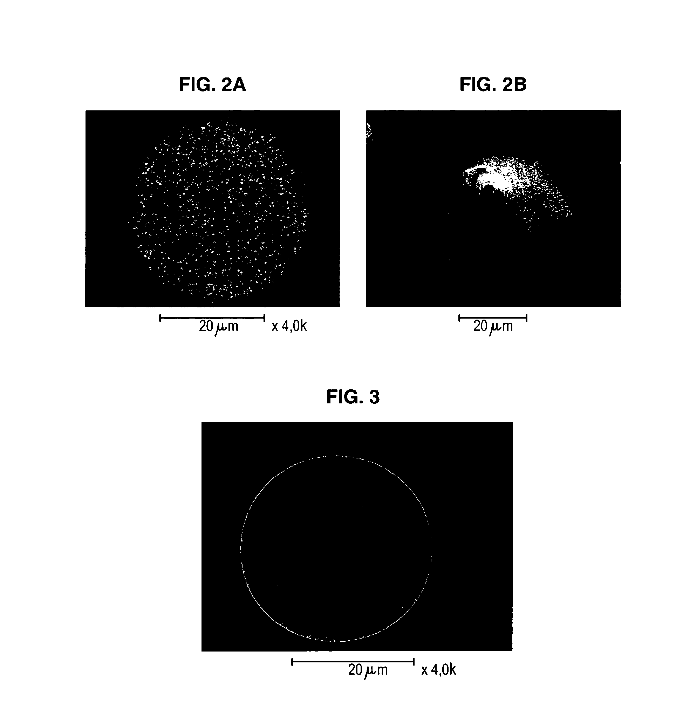

Arrays of gold or platinum first microelectrodes were modified using macroporous, and mesoporous approaches, respectively. Macroporous electrodes had pore diameters of about 500 nm (Figures 2A and 2B), while mesoporous microelectrodes had pore diameters within 1-10 nm (Figure 3 for the example of a 32 $\mu$m diameter mesoporous micro-lelectrode).

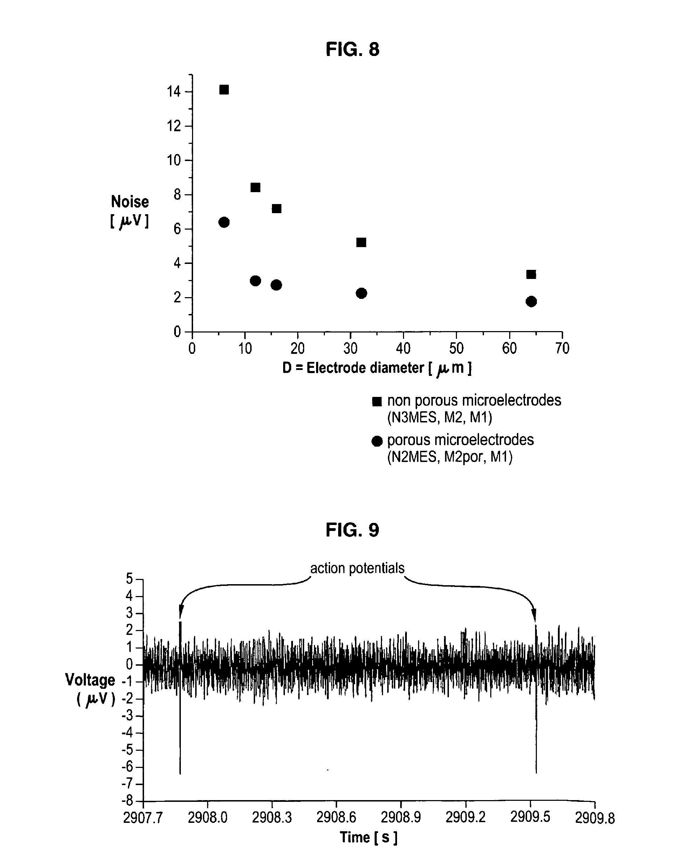

Examples of noise reduction using mesoporous modification are given in Figure 8 showing examples of noise level reduction in ordinate using mesoporous modification for different sizes of zone 11 of the first microelectrode between 28 and 3200 $(\mu m)^2$ in abscissa, with a zone 11 being a plane disk of diameter D according to Figure 1A. The first underlying material of the geometric surface of electrode and zone 11 are made of platinum with a porous layer of platinum, i.e. M1 = Pt, M2por =Pt and M2 = Pt in the example of Figure 8. In this case, the geometric surface of zone 11 for application against the living tissue is a disk of diameter D, whose geometric area S is equal to $\pi . D^2/4$. The noise level of the instrumentation serving to measure the noise was 1 $\mu$V. Consequently, on Figure 8, the intrinsic RMS voltage noise level of the electrode having zone 11 for application against the living tissue has to be corrected.

Here are for different values of the diameter D of zone 11 for application against the living tissue:

- the measured standard deviation N2MES of the recording voltage noise level, indicated by a disk on Figure 8 for the first porous electrode having the first geometric surface of zone 11 of diameter D for application against the living tissue, wherein the first geometric surface of said first porous electrode is formed by the second external porous layer made of the second porous material M2por,

- the corrected standard deviation $N_{M2por}$ of the recording voltage noise level, which is obtained by a correction of the measured standard deviation N2MES of the recording voltage noise level, and which is equal to the standard deviation of intrinsic recording voltage noise $N_{M2por}$ measured for the first porous electrode having the first geometric surface of zone 11 of diameter D for application against the living tissue, wherein the first geometric surface of said first porous electrode is formed by the second external porous layer made of the second porous material M2por :

$$N_{M2por} = ((N2MES)^2 - 1)^{1/2} .$$

- the measured standard deviation N3MES of the recording voltage noise level N3MES indicated by a square on Figure 8 for said second non porous comparison electrode, which is another electrode which has been fabricated with the same first geometric surface of zone 11 of diameter D for application against the living tissue, wherein said other second comparison electrode is the same as the first electrode 10 but has not the geometric surface of its zone for application to the living tissue formed by the porous layer consisting in the second porous material M2por, wherein the first geometric surface of the zone for application to the living tissue of said other second comparison electrode is formed of said at least one second material M2 being non porous;

- the corrected standard deviation $N_{M2}$ of the recording voltage noise level N3MES for the second non porous comparison electrode, which is obtained by a correction of the measured standard deviation N3MES of the recording voltage noise level N3MES, and which is equal to the standard deviation of the intrinsic recording voltage noise $N_{M2}$ for the second non porous comparison electrode, which is said other electrode having the same first geometric surface of zone 11 of diameter D for application against the living tissue, wherein said other second comparison electrode is the same as the first electrode 10 but has not the geometric surface of its zone for application to the living tissue formed by the porous layer consisting in the second porous material M2por, wherein the first geometric surface of the zone for application to the living tissue of said other second comparison electrode is formed of said at least one second material M2 being non porous :

$$N_{M2} = ((N3MES)^2 - 1)^{1/2}$$

| Diameter D ($\mu$m) | N3MES (non porous), $\mu$V | Intrinsic $N_{M2}$ (non porous), $\mu$V | N2MES (porous), $\mu$V | Intrinsic $N_{M2por}$ (porous), $\mu$V |
|---|---|---|---|---|
| 6 | 14.13 | 14.09 | 6.39 | 6.31 |
| 12 | 8.40 | 8.34 | 2.99 | 2.82 |
| 16 | 7.19 | 7.12 | 2.76 | 2.57 |
| 32 | 5.22 | 5.12 | 2.25 | 2.02 |
| 64 | 3.32 | 3.16 | 1.78 | 1.47 |

In a general manner, for a first geometric surface of zone 11 of any prescribed shape, the value D is called the width of the zone 11 for application against the living tissue and is calculated by the following second formula

$$D = (4.S/\pi)^{1/2}$$

wherein S is the area of the geometric surface of the zone 11,
wherein the second porous layer is situated on said first geometric surface of the zone 11. Then, the zone 11 can have any geometric surface, for example rectangular or square or any shape in a plane, but also non plane shapes, like for example conic or others.
When the first geometric surface of zone 11 has the second porous external layer, the second porous external layer has a developed surface area DSA which is proportional to an area ADSA measured on the voltamogram of the electrode, as described below. The developed surface area DSA of the second porous external layer of the first electrode is higher than the area of its first geometric surface.
Electrochemical characterization of macroporous and mesoporous first electrodes:

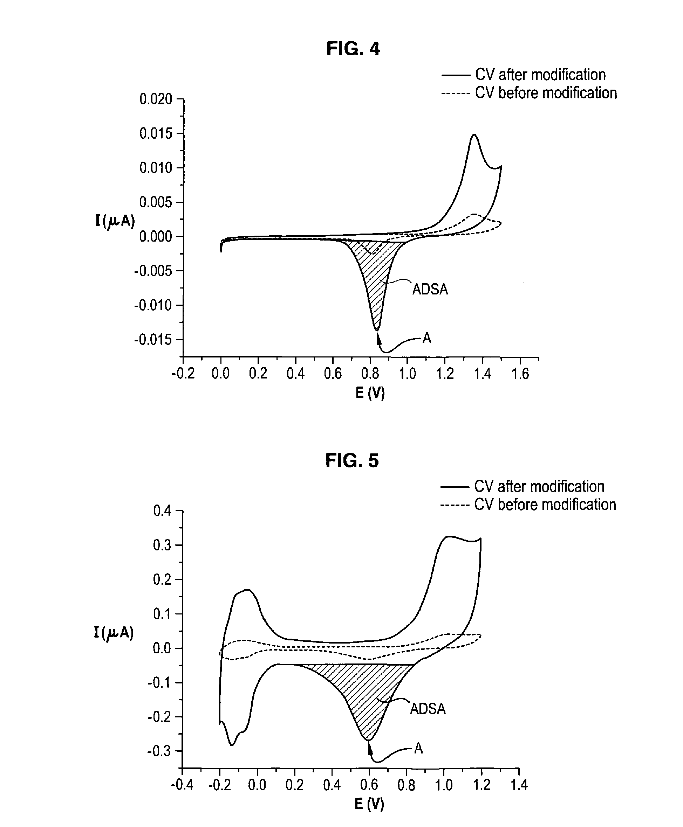

Microelectrodes were characterized using cyclic voltametry before and after macroporous or mesoporous modifications. In the example of figure 4, macroporous modification of gold microelectrodes led to an increase of the active surface by a factor of 6, with M1 =Au, M2por = Au and M2 = Au. In the example of figure 5, mesoporous modification of a platinum microelectrode with M1 =Pt, M2por =Pt and M2 = Pt leads to an increase of the active surface by more than an order of magnitude, wherein n orders of magnitude are equal to $10^n$.

Figure 4 shows a cyclic voltamogram (CV) of the current in ordinate depending on voltage E in abscissa for a gold microelectrode, before the porous modification (interrupted line, second electrode) and after the macroporous modification (continuous line, first electrode).
Figure 5 shows a cyclic voltamogram (CV) of the current in ordinate depending on voltage E in abscissa for a platinum microelectrode, before the porous modification (interrupted line, second electrode) and after the mesoporous modification (continuous line, first electrode).
The voltamogram of the porous electrode has an abyss A whose area ADSA on the voltamogram is proportional to the developed surface area DSA of zone 11. Said abyss A reaches much higher absolute values than the corresponding voltamogram for the non porous electrode of same width D. The developed surface area DSA of zone 11 is determined from the voltamogram and enables to verify the porosity of the porous layer, in order to have the required noise level for width D.
A method to constitute the first electrode can be the following.
In order to have a prescribed noise N3MES, the corresponding width D is determined on figure 8 according to equation
Log (N3MES) = 1.6 - 0.6 log (D),
to obtain the width D of the non porous second electrode.
An equivalent non porous geometric surface area S of the zone 11 for application to a living tissue is determined by

$S = \pi.D^2/4$.

Then the value of the developed surface area DSA of the porous layer that should be brought on the first geometric surface for the first electrode is rendered equal to S

$DSA = S$.

Then the voltamogram of the first electrode, on the zone 11 of which the porous layer has been brought, is determined to have an area ADSA of abyss A corresponding to the calculated value of the developed surface area DSA to determine the features to impose to the porous layer based on ADSA.

Noise reduction for neural signal recording of the first electrode:

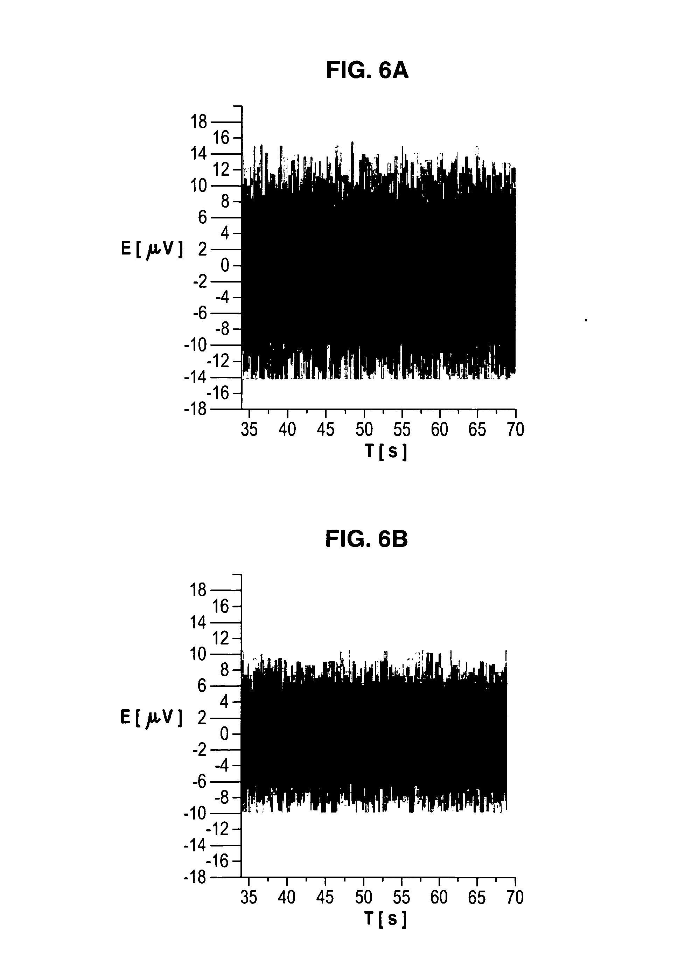

Macroporous modification leads to a reduction of noise level by a factor up to about 1.5 for a 40-$\mu$m gold microelectrode (Figures 6A and 6B).

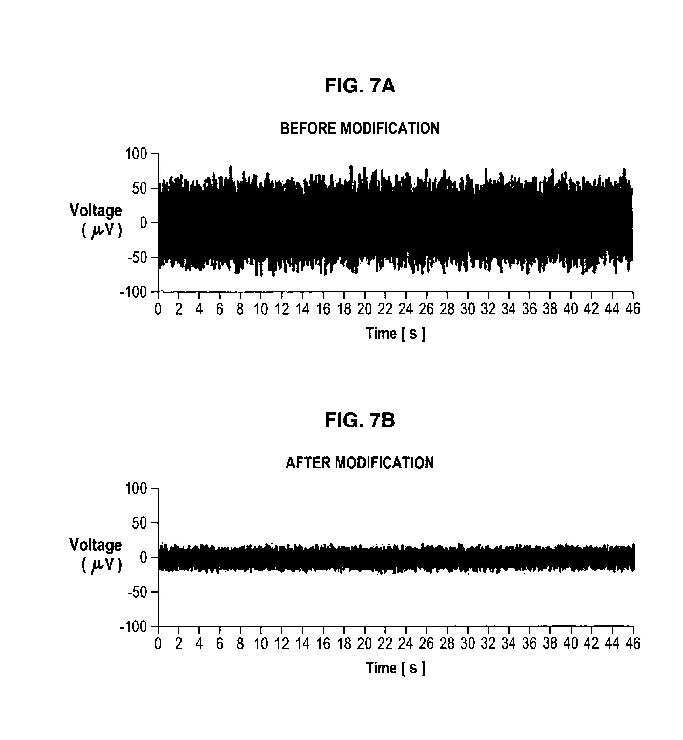

Mesoporous modification leads to a stronger reduction of noise by factors of typically x3 for a 12-$\mu$m diameter planar platinum electrode, as shown in Figures 7A and 7B.

Figure 6A shows the signal voltage in ordinate depending on time in abscissa before the porous modification (second electrode) and figure 6B shows the signal voltage in ordinate depending on time in abscissa after the porous modification for a macroporous modification (first electrode).

Figure 7A shows the signal voltage in ordinate depending on time in abscissa before the porous modification (second electrode) and figure 7B shows the signal voltage in ordinate depending on time in abscissa after the porous modification (first electrode) for a mesoporous modification of a 12-$\mu$m-diameter microelectrode.

Porous electrode modification yields 2 advantages for neural stimulation. First, the increased active surface of first microelectrodes allows the injection of higher currents while remaining in the reversible domain and thus without electrode and tissue damage. In a subsidiary manner, the increased active surface of the supplementary third conductive surface 3 yields a more focal stimulation.

The present invention allows solving the following problems for recording or stimulating electrogenic cells:

Reduction of noise level using microelectrodes or macroelectrodes for recording electrical signals originating from electrogenic cells either in vitro or in vivo, e.g.: action potentials, local field potentials, scalp electroencephalography (EEG), electrocorticograms (ECoG) using epi- or sub-dural electrodes, stereotaxic EEG (SEEG) using implanted macroscopic electrodes;

Higher current injection capability using microelectrodes or macroelectrodes either in vitro or in vivo;

Higher stimulation focality of electrical stimulation using the approach of a supplementary second conductive surface surrounding the electrodes of an array;

Increasing active surface of microelectrodes for electrogenic cell recording and stimulation;

Increasing active surface of macroelectrodes for electrogenic cell recording and stimulation;

Increasing active surface of the supplementary conductive section for increased focality of stimulations.

The field of application of the invention comprises :

Exploration of neural networks in vitro or in vivo using microelectrodes or macroelectrodes and arrays of such, like microelectrode arrays (MEAs), EEG, SEEG, or ECoG;

Development of neural implants for functional rehabilitation (e.g., cochlear, retinal, spinal cord, cortical, sub-cortical, hippocampic implants);

Exploration of other types of electrogenic cells, such as cardiac cells, muscles, vessel cells, stem cells, pancreatic cells;

Development of cardiac implants for recording of physiological signals or stimulation of the heart, part of the heart, or part of the cardio-vascular system.

In an embodiment, the geometrical surface of zones 11 of first microelectrodes is greater than 0.1 $(\mu m)^2$ and smaller than 100 $(\mu m)^2$.

In an embodiment, the geometrical surface of zones 11 of first microelectrodes is greater than 0.1 $(\mu m)^2$ and smaller than 400 $(\mu m)^2$.

In an embodiment, the first microelectrodes 10 may be bi-dimensional or three-dimensional.

In an embodiment, the first microelectrodes 10 may be microwire sections or parts of a microwire's surface.

In an embodiment, the geometrical surface of first macroelectrodes 10 is greater than 50000 $(\mu m)^2$ and smaller than 10 cm$^2$.

In an embodiment, the thickness of the porous layer is between 1 nm and 10 $\mu$m

Electrode materials M1 can be: Pt, Au, Ir, Ir oxyde, TiN, Tungstene, ITO, semiconductors, alloys, conducting polymers or any other conducting material.

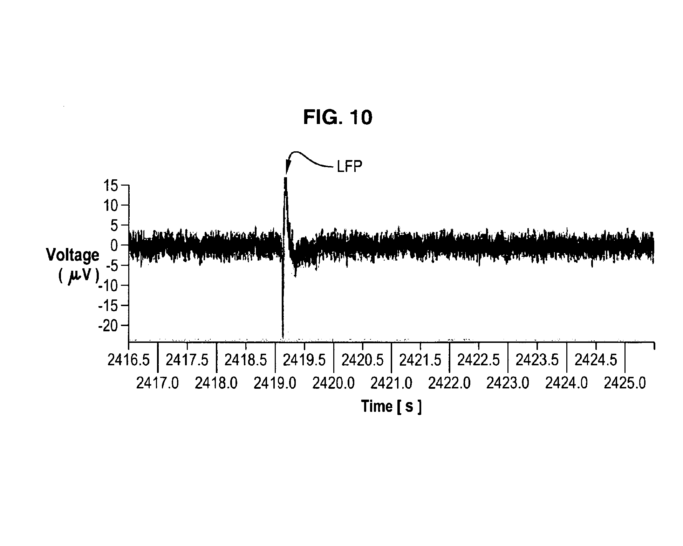

A modified first microelectrode array (MEA) was used for the recording of the activity of an embryonic mouse hindbrain and spinal cord in order to show its application for real measurements. Immediately after dissection of the mouse embryo, the neural tissue was put onto the electrode sites of the MEA that was filled with Ringer solution. In this structure, two types of neural signals were recorded using mesoporous platinum microelectrodes of 30 $\mu$m diameter: action potentials (Figure 9 showing voltage in ordinate depending on time in abscissa) and local field potential (Figure 10 showing voltage in ordinate depending on time in abscissa with measurement of local field potential LFP).

The improved electro-chemical properties of the modified array contribute to lower noise content compared to the non-modified one and thus the recordings of extracellular signals can benefit from the apparent improvement of signal-to-noise ratio (SNR), since the quality of the recorded extracellular signals can be evaluated in terms of their SNR characteristics, the overall signal amplitude and the shape of the signal. The better recording performances of the modified electrodes allow to see cellular events that otherwise would have been hidden by the noise and we can therefore conclude on an overall increase in the performance of the recording or stimulating system.

Below are shown example of RMS noise level values before (second electrode) and after mesoporous modification (first electrode) for three electrodes of different diameter 2 $\mu$m, 32 $\mu$m and 64 $\mu$m:

| Diameter ($\mu$m) - | RMS before ($\mu$V) - | RMS after ($\mu$V) - | Factor of reduction |
|---|---|---|---|
| 2 | 17.83 | 4.71 | 3.8 |
| 32 | 6.73 | 2.22 | 3 |
| 64 | 4.36 | 2.79 | 1.6 |

From the resulting RMS values one can see that the highest noise reduction factor was obtained for the electrode with the smallest diameter (2 $\mu$m) and the lowest noise reduction was obtained for the biggest electrode (64 $\mu$m).

The noise measurements of the figures were performed in a Ringer solution composed of (in mM): 113 NaCl, 4.5 KCl, 2 $CaCl_2 2H_2O$, 1 $MgCl_2 6H_2O$, 25 $NaHCO_3$, 1 $NaH_2PO_4 H_2O$ and 11 D-Glucose.

## Claims

1. Device to stimulate or record a signal to or from a living tissue, comprising at least one electrically conductive first electrode (10) and/or supplementary conductive surface (3) comprising a zone (11) for application to the living tissue and a conductor for sending or receiving a signal voltage to or from the zone (11), wherein the zone (11) for application to the living tissue is connected to said conductor,

   **characterized in that**
   the zone (11) for application to the living tissue belonging to the first electrode (10) and/or supplementary conductive surface (3) has a first geometric surface formed of an external porous layer consisting in at least one second material (M2por) being porous and arranged in such a manner that the first electrode has a standard deviation of intrinsic recording voltage noise $N_{M2por}$ measured under defined conditions according to the following first formula
   $N_{M2por} \leq 0.9\ N_{M2}$ ,
   wherein $N_{M2}$ is the standard deviation of the intrinsic recording voltage noise measured under the same defined conditions with another second comparison electrode and/or supplementary conductive surface being the same as the first electrode having its zone for application to the living tissue having the same first geometric surface but formed of said at least one second material (M2) being non porous,
   wherein said conditions comprise measuring the voltage in a bandwith between 1 and 3000 Hz in a physiological liquid at 25 °C.

2. Device according to claim 1, **characterized in that** the external porous layer consisting in said at least one second material (M2$_{por}$) being porous of the first geometric surface of the first electrode (10) is formed on a first underlying material (M1) of the zone (11) for application to the living tissue connected to said conductor.

3. Device according to claim 1, **characterized in that** according to the second following formula
   $\log(N_{M2por}) \leq 1.5 - 0.57 \log(D)$,
   wherein
   log is the decimal logarithm,
   D is a width of the zone (11) of the first electrode in $\mu$m,
   N is in $\mu$Y.

4. Device according to claim 1, **characterized in that** according to the third following formula
log ($N_{M2por} \leq 1.5 - 0.6$ log (D), wherein
log is the decimal logarithm,
D is a width of the zone (11) of the first electrode in $\mu$m,
N is in $\mu$Y.

5. Device any one of the claims 3 and 4, **characterized in that** the width D is the diameter of the zone (11) having the first geometric surface being circular.

6. Device according to any one of the claims 3 to 5, **characterized in that** the width D is calculated by the following fourth formula
$D = (4.S/\pi)^{1/2}$
wherein S is the area of the first geometric surface of the zone (11).

7. Device according to any one of the claims 3 to 6, **characterized in that**
$1 \mu m \leq D \leq 100 \mu m$.

8. Device according to any one of the claims 1 to 7, **characterized in that** the geometric area of the first geometric surface of the zone (11) for local application to the living tissue is greater than 0.1 $(\mu m)^2$ and smaller than 400 $(\mu m)^2$.

9. Device according to any one of the claims 1 to 7, **characterized in that** the geometric area of the first geometric surface of the zone (11) for local application to the living tissue is greater than 50000 $(\mu m)^2$ and smaller than 10 cm$^2$.

10. Device according to any one of the claims 1 to 7, **characterized in that** the geometric area of the first geometric surface of the zone (11) for local application of the supplementary conductive surface (3) to the living tissue is greater than 100 $(\mu m)^2$ and smaller than 1000 cm$^2$.

11. Device according to any one of the preceding claims, **characterized in that** the standard deviation of intrinsic recording voltage noise $N_{M2por}$ of the first electrode is lower than 3 $\mu$V for the zone (11) for local application to the living tissue having a width D between 12 $\mu$m and 64 $\mu$m

12. Device according to any one of the preceding claims, **characterized in that** the porous layer has a thickness between 1 nm and 10 $\mu$m

13. Device according to any one of the preceding claims, **characterized in that** the porous layer has a uniform pore size.

14. Device according to any one of the preceding claims, **characterized in that** the external porous layer consisting in said at least one second material (M2por) being porous of the first geometric surface of the first electrode (10) is formed on a first underlying material (M1) of the zone (11) for application to the living tissue connected to said conductor, wherein the at least one first material (M1) is a conductive material which is the same as the at least one second material (M2).

15. Device according to any one of the preceding claims, **characterized in that** the external porous layer consisting in said at least one second material (M2por) being porous of the first geometric surface of the first electrode (10) is formed on a first underlying material (M1) of the zone (11) for application to the living tissue connected to said conductor, wherein the at least one first material (M1) is a conductive material which is different from the at least one second material (M2).

16. Device according to any one of the preceding claims, **characterized in that** the external porous layer is made of Pt, Au, Ir, TiN, Ir oxide, Tungstene, ITO, semiconductors, alloys, conducting polymers or any other conducting material.

17. Device according to any one of the preceding claims, **characterized in that** the first electrode (10) is a first microelectrode and an array (1) of first microelectrodes (11) is provided, wherein the array (1) of first microelectrodes (11) is arranged in a determined configuration side-by-side, wherein each first microelectrode can be selected, the first microelectrodes (11) being insulated from each other, the conductors being insulated from each other, the zones (11) for local application to the living tissue being insulated from each other.

**18.** Device according to claim 13, **characterized in that** a plurality of third conductive zones (13) are located, respectively for a determined plurality of first microelectrodes (10), in the vicinity of the plurality of zones (11) of said determined first microelectrodes (11) of the array and are insulated from these zones (11) for application to the living tissue, said plurality of conductive third zones (13) belongs to a third conductive surface (3) supplemental to the first microelectrodes (11) and separate from the first microelectrodes (11), the third supplemental conductive surface (3) being used for all or part of the application against the living tissue, connection means being provided to ensure electric connection between said plurality of third conductive zones (13) of the supplemental third surface (3) for all said determined plurality of first microelectrodes (10) insulated from one another, so that the third conductive zones (31) of the third supplemental surface (3) are substantially equipotential, the supplemental third conductive surface (3) also being connected to at least one port (33) intended to be connected to an external return conductor (35) returning at least part of the signal and being formed to ensure focal stimulation from at least one of the determined plurality of first microelectrodes (10) and to act as common focusing means for several zones (11) of different determined first microelectrodes (10).

**19.** Device according to any one of the preceding claims, **characterized in that** the physiological liquid is a Ringer's solution.

**20.** Device according to any one of the preceding claims, **characterized in that** the physiological liquid is a Ringer's solution composed of, in mM,: 113 NaCl, 4.5 KCl, 2 $CaCl_2 2H_2O$, 1 $MgCl_2 6H_2O$, 25 $NaHCO_3$, 1 $NaH_2PO_4 H_2O$ and 11 D-Glucose.

## FIG. 1A

10, 11, M2por

1

L

## FIG. 1B

10, 11, M2por

1

L

**FIG. 1C**

10, 11, M2por

4

4

4

4

1

L

10, 11, M2por

10, 11, M2por

10, 11, M2por    10, 11, M2por

**FIG. 1D**

4    4    4    4    4    1

4    4

L

10, 11, M2por

10, 11, M2por

10, 11, M2por

10, 11, M2por

**FIG. 1E**

L ↓

12

1

12

12

12

12

10, 11, M2por

**FIG. 1F**

L ↓

11, M2por    11, M2por    11, M2por    10    1    5

**FIG. 1G**

L ↓

10, 11, M2por    1    12

**FIG. 1H**

L ↑

13    10, 11, M2por    13    13    1    10, 11, M2por

35    33

10, 11, M2por

13

**FIG. 2A**

**FIG. 2B**

20 μm | x 4,0k

20 μm

**FIG. 3**

20 μm | x 4,0k

**FIG. 4**

**FIG. 5**

## FIG. 6A

## FIG. 6B

## FIG. 7A

### BEFORE MODIFICATION

## FIG. 7B

### AFTER MODIFICATION

**FIG. 8**

■ non porous microelectrodes
(N3MES, M2, M1)

● porous microelectrodes
(N2MES, M2por, M1)

**FIG. 9**

FIG. 10

Europäisches
Patentamt

European
Patent Office

Office européen
des brevets

## EUROPEAN SEARCH REPORT

**Application Number**

EP 10 30 5609

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2009/053333 A1 (CENTRE NAT RECH SCIENT [FR]; GROUPE ECOLE SUPERIEURE D INGE [FR]; UNIV) 30 April 2009 (2009-04-30) <br> * abstract * <br> * page 1, lines 1-28 * <br> * page 3, lines 17-34 * <br> * page 14, lines 26-36 * <br> ----- | 1-20 | INV. <br> A61N1/36 <br> A61N1/05 |

TECHNICAL FIELDS
SEARCHED (IPC)

A61N

The present search report has been drawn up for all claims

| Place of search | Date of completion of the search | Examiner |
|---|---|---|
| Munich | 29 October 2010 | Pfeiffer, Uwe |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

 

& : member of the same patent family, corresponding
   document

EPO FORM 1503 03.82 (P04C01)

**ANNEX TO THE EUROPEAN SEARCH REPORT
ON EUROPEAN PATENT APPLICATION NO.**

EP 10 30 5609

This annex lists the patent family members relating to the patent documents cited in the above-mentioned European search report.
The members are as contained in the European Patent Office EDP file on
The European Patent Office is in no way liable for these particulars which are merely given for the purpose of information.

29-10-2010

| Patent document cited in search report | Publication date | Patent family member(s) | | Publication date |
|---|---|---|---|---|
| WO 2009053333 A1 | 30-04-2009 | CA | 2702277 A1 | 30-04-2009 |
| | | EP | 2217322 A1 | 18-08-2010 |
| | | FR | 2922460 A1 | 24-04-2009 |

EPO FORM P0459

**EP 2 394 696 A1**

## REFERENCES CITED IN THE DESCRIPTION

**Patent documents cited in the description**

- WO 2009053333 A

- US 6503382 B